(19) **Europäisches Patentamt / European Patent Office / Office européen des brevets**

(11) **EP 4 343 659 B1**

(12) **EUROPEAN PATENT SPECIFICATION**

(45) Date of publication and mention
of the grant of the patent:
**22.10.2025 Bulletin 2025/43**

(21) Application number: **23192459.8**

(22) Date of filing: **21.08.2023**

(51) International Patent Classification (IPC):
**G06Q 30/0201** (2023.01)　　**G06Q 30/0283** (2023.01)
**G06Q 40/08** (2012.01)

(52) Cooperative Patent Classification (CPC):
**G06Q 30/0201; G06Q 30/0206; G06Q 30/0283;
G06Q 40/08**

(54) **METHOD AND SYSTEM FOR DECISION SUPPORT IN PHARMACEUTICAL PRICING**

VERFAHREN UND SYSTEM ZUR ENTSCHEIDUNGSUNTERSTÜTZUNG IN DER
PHARMAZEUTISCHEN PREISGESTALTUNG

PROCÉDÉ ET SYSTÈME D'AIDE À LA DÉCISION DANS LE PRIX PHARMACEUTIQUE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **23.09.2022 IN 202221054667**

(43) Date of publication of application:
**27.03.2024 Bulletin 2024/13**

(73) Proprietor: **Tata Consultancy Services Limited
Maharashtra (IN)**

(72) Inventors:
  • **THIRUNAVUKKARASU, Jeisobers
    600096 Chennai, Tamil Nadu (IN)**

  • **RAO, Shilpa Yadukumar
    600096 Chennai, Tamil Nadu (IN)**
  • **GOPAL, Dhanasekaran
    600096 Chennai, Tamil Nadu (IN)**

(74) Representative: **Goddar, Heinz J.
Boehmert & Boehmert
Anwaltspartnerschaft mbB
Pettenkoferstrasse 22
80336 München (DE)**

(56) References cited:
**US-A1- 2014 358 578　　US-A1- 2020 105 392**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

CROSS-REFERENCE TO RELATED APPLICATIONS AND PRIORITY

The present application claims priority to Indian application no. 202221054667, filed on September 23, 2022.

TECHNICAL FIELD

The disclosure herein generally relates to decision support system for pharmaceutical pricing and, more particularly, to a method and system for providing visibility on nature of pricing followed by different entities of pharma players associated with retail pharmacy and to enable enhanced decision making related with the pharma players.

BACKGROUND

Retail pharmacy is facing challenges in choosing pharma players such as pharmacy benefit managers (PBMs), distributor, and so on, who can serve specific requirements, as there is no easier way of understanding of pricing strategies of pharma players. For the same reason, patients face challenges in choosing entities or pharma players such as insurer, insurance plans and brand of drugs, and pharmacy benefit managers. Many players are involved in pharma value chain i.e., from manufacturing to usage by patient and each player adds price variations which causes challenges in traceability with respect to price variations for a drug and across drugs. It is challenging to model price of a drug due to difficulties in identifying the causes for the price variation across drugs and each drug has specific chemical composition with no direct or exact relation with price of a drug. In pharmaceutical industry, price of a drug is not dependent on the quantity of drugs sold which is unique as compared to retail industry.

Traditional approaches in this domain face challenges with respect to traceability of pricing behavior by pharma players such as pharmacy benefit managers (PBMs), distributor, and so on, and hence fail to provide visibility on nature of pricing followed by different entities of pharma players.

Document US20140358578A1 discloses a system and method for comparing pharmaceutical prices and mediation utilization that provides separate databases containing (i) client pharmaceutical wholesaler and non-wholesaler purchase data, (ii) pharmaceutical compendia information, (iii) client pharmaceutical contract information, (iv) client organizational profile information, and (v) client patient volume and acuity data; and a processor in communication with the databases that (i) uploads the data and information from the databases into an input module that standardizes, validates and merges the data and information, (ii) processes the data and information from the input module by performing pricing and utilization analyses on the information and generating savings opportunities information, and (iii) formats the savings opportunities information and generates and pricing and utilization reports. A database interface for customized reporting and research analytics is also provided.

Document US 2020/105392 AI discloses Methods, systems, and techniques for real-time medication pricing, optimizing purchase options, analyzing medication conflicts, and optimizing medical provider options are provided. Example embodiments provide a Healthcare Advisory System ("HAS"), which enables users to select their optimal medication purchase options and enables discount drug provider ("DDPs") to capture or retain sales that they otherwise would have lost due to price competition. In one embodiment, the HAS includes a HAS server, drug supply chain intermediary systems, pharmacy benefit manager systems, insurance provider systems, and client devices. These components cooperate to reduce computational expensive trend-based analytics that DDPs otherwise would employ to achieve worse results, thereby improving the computational efficiency of the enhanced computer- and network-based methods, techniques, and systems.

SUMMARY

Embodiments of the present disclosure present technological improvements as solutions to one or more of the above-mentioned technical problems recognized by the inventors in conventional systems. The invention is set out in the appended set of claims.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

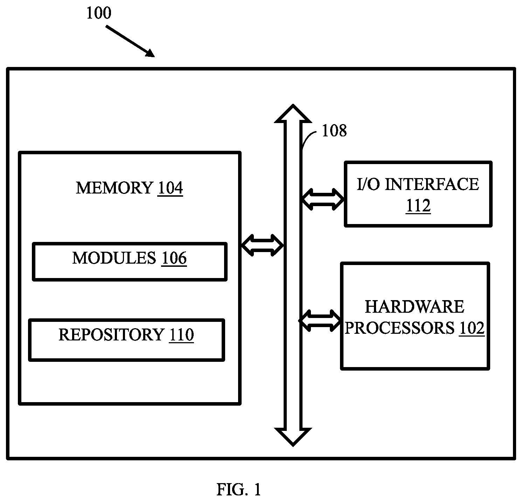

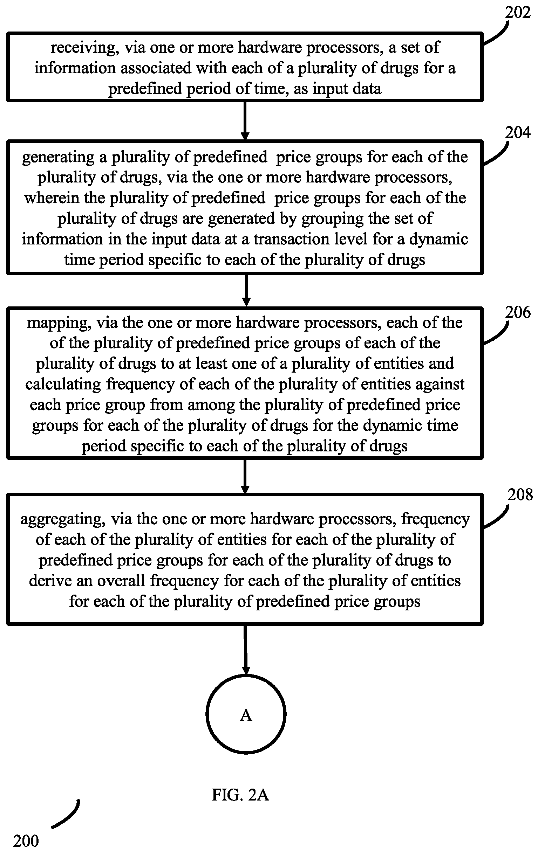

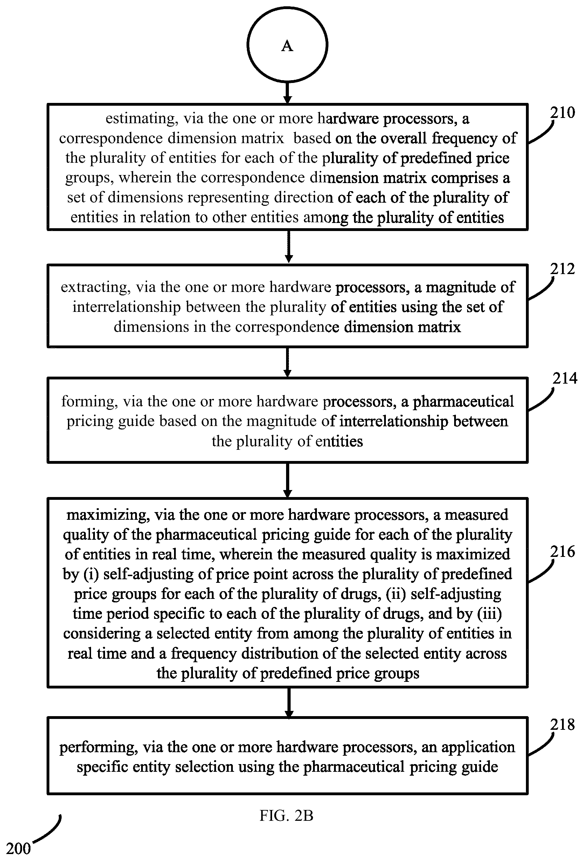

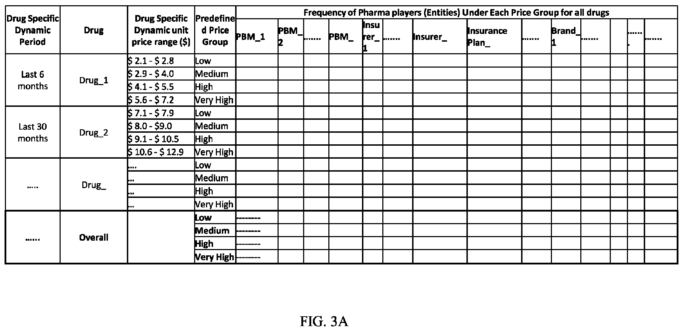

FIG. 1 illustrates an exemplary system for pricing analysis, according to some embodiments of the present disclosure. FIGS. 2A and 2B (collectively referred to as FIG. 2) is a flow diagram depicting steps involved in the process of the pricing analysis performed by the system of FIG. 1, according to some embodiments of the present disclosure. FIGS. 3A through 3H depict example of the pricing analysis being carried out by the system of FIG. 1, in accordance with some embodiments of the present disclosure.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

Traditional approaches used for pricing analysis in pharmaceutical domain face challenges with respect to traceability of pricing behavior by pharma players such as pharmacy benefit managers (PBMs), distributor, and so on, and hence fail to provide visibility on nature of pricing followed by different entities of pharma players.

Embodiments herein disclose a method and system for decision support for pharmaceutical pricing. The system, by performing the pricing analysis, extracts a magnitude of interrelationship between the plurality of entities in the pharmaceutical domain, and forms a pharmaceutical pricing guide based on the magnitude of interrelationship between the plurality of entities. The pharmaceutical pricing guide is further processed to maximize a measured quality of the pharmaceutical pricing guide for each of the plurality of entities in real time. The pharmaceutical pricing guide is further used for application specific entity selection.

Referring now to the drawings, and more particularly to FIG. 1 through FIG. 3H, where similar reference characters denote corresponding features consistently throughout the figures, there are shown preferred embodiments and these embodiments are described in the context of the following exemplary system and/or method.

FIG. 1 illustrates an exemplary system for pricing analysis, according to some embodiments of the present disclosure. The system 100 includes or is otherwise in communication with hardware processors 102, at least one memory such as a memory 104, an I/O interface 112. The hardware processors 102, memory 104, and the Input /Output (I/O) interface 112 may be coupled by a system bus such as a system bus 108 or a similar mechanism. In an embodiment, the hardware processors 102 can be one or more hardware processors.

The I/O interface 112 may include a variety of software and hardware interfaces, for example, a web interface, a graphical user interface, and the like. The I/O interface 112 may include a variety of software and hardware interfaces, for example, interfaces for peripheral device(s), such as a keyboard, a mouse, an external memory, a printer and the like. Further, the I/O interface 112 may enable the system 100 to communicate with other devices, such as web servers, and external databases.

The I/O interface 112 can facilitate multiple communications within a wide variety of networks and protocol types, including wired networks, for example, local area network (LAN), cable, etc., and wireless networks, such as Wireless LAN (WLAN), cellular, or satellite. For the purpose, the I/O interface 112 may include one or more ports for connecting several computing systems with one another or to another server computer. The I/O interface 112 may include one or more ports for connecting several devices to one another or to another server.

The one or more hardware processors 102 may be implemented as one or more microprocessors, microcomputers, microcontrollers, digital signal processors, central processing units, node machines, logic circuitries, and/or any devices that manipulate signals based on operational instructions. Among other capabilities, the one or more hardware processors 102 is configured to fetch and execute computer-readable instructions stored in the memory 104.

The memory 104 may include any computer-readable medium known in the art including, for example, volatile memory, such as static random-access memory (SRAM) and dynamic random-access memory (DRAM), and/or non-volatile memory, such as read only memory (ROM), erasable programmable ROM, flash memories, hard disks, optical disks, and magnetic tapes. In an embodiment, the memory 104 includes a plurality of modules 106.

The plurality of modules 106 include programs or coded instructions that supplement applications or functions performed by the system 100 for executing different steps involved in the process of the pricing analysis, being performed by the system 100. The plurality of modules 106, amongst other things, can include routines, programs, objects, components, and data structures, which performs particular tasks or implement particular abstract data types. The plurality of modules 106 may also be used as, signal processor(s), node machine(s), logic circuitries, and/or any other device or component that manipulates signals based on operational instructions. Further, the plurality of modules 106 can be used by hardware, by computer-readable instructions executed by the one or more hardware processors 102, or by a combination thereof. The plurality of modules 106 can include various sub-modules (not shown). The plurality of modules 106 may include computer-readable instructions that supplement applications or functions performed by the system 100 for the pricing analysis.

The data repository (or repository) 110 may include a plurality of abstracted piece of code for refinement and data that is processed, received, or generated as a result of the execution of the plurality of modules in the module(s) 106.

Although the data repository 110 is shown internal to the system 100, it will be noted that, in alternate embodiments, the data repository 110 can also be implemented external to the system 100, where the data repository 110 may be stored within a database (repository 110) communicatively coupled to the system 100. The data contained within such external database may be periodically updated. For example, new data may be added into the database (not shown in FIG. 1) and/or existing data may be modified and/or non-useful data may be deleted from the database. In one example, the data may be stored in an external system, such as a Lightweight Directory Access Protocol (LDAP) directory and a Relational Database Management System (RDBMS). Functions of the components of the system 100 are now explained with reference to the steps in flow diagram in FIG. 2, and the example diagrams in FIG. 3A through 3H.

FIGS. 2A and 2B (collectively referred to as FIG. 2) is a flow diagram depicting steps involved in the process of the pricing analysis performed by the system of FIG. 1, according to some embodiments of the present disclosure.

Steps in the method 200 are explained with reference to the components of the system 100 as depicted in FIG. 1. In an embodiment, the system 100 comprises one or more data storage devices or the memory 104 operatively coupled to the processor(s) 102 and is configured to store instructions for execution of steps of the method 200 by the processor(s) or one or more hardware processors 102. The steps of the method 200 of the present disclosure will now be explained with reference to the components or blocks of the system 100 as depicted in FIG. 1 and the steps of flow diagram as depicted in FIG. 2. Although process steps, method steps, techniques or the like may be described in a sequential order, such processes, methods, and techniques may be configured to work in alternate orders. In other words, any sequence or order of steps that may be described does not necessarily indicate a requirement that the steps to be performed in that order. The steps of processes described herein may be performed in any order practical. Further, some steps may be performed simultaneously.

At step 202 of the method 200, the system 100 receives via the one or more hardware processors 102, a set of information associated with each of a plurality of drugs for a predefined period of time, as input data. The input data may include data pertaining to each drug, collected over a predefined period of time, and is retrieved from various databases such as but not limited to pharmacy Point Of Sale (POS), databases having information with associated pharmacy benefit managers (PBMs), insurer, insurance plans, and drug information such as but not limited to brand, form, manufacturer, and distributor.

Further, at step 204 of the method 200, a plurality of predefined price groups are generated for each of the plurality of drugs, via the one or more hardware processors 102. The plurality of predefined price groups for each of the plurality of drugs are generated by grouping the set of information in the input data at a transaction level for a dynamic time period specific to each of the plurality of drugs. The term 'dynamic time period' refers to a particular time period that has been opted by the user, and information corresponding to this particular time period, from the input data, forms the set of information that is grouped by the system 100. The system 100 may be configured to consider each row of a prescription as a transaction, and may uniquely identify each transaction using a key formed by combining a bill number and row number. The key may be formed by creating uniqueness in different ways within the scope of present disclosure. Price of a drug for each transaction is processed to derive measures such as unit price, to enable comparison across formats and drugs. For example, tablet, syrup are some forms of a drug. In one instance price of a unit of the drug is calculated, wherein the unit may be chemical amount in grams (gms) or milliliter (ml) or in any other suitable quantity. The derived unit price at transaction level of a time period of a drug is used to form a price group which is specific to that drug. Here the time period varies depending on the drug and the time period is dynamic based on the outcomes which is explained in the later sections. The grouping may be done in many ways within the scope pf present disclosure. In one instance distribution of unit prices is considered and four groups are formed by sorting of prices and named as group with low, medium, high, and very high prices. In another instance, clustering of unit prices is done using a k-means algorithm in which value of k maybe fixed as 4 and based on the group means or average value of each group, they are noted as low, medium, high and very high. Group means or average value of each group is used to decide adjacency. For example, low and medium price groups are considered as adjacent price groups. The price group is formed separately for each drug, and thus the plurality of predefined price groups are generated. The process of grouping of prices enables standardization of pricing across drugs as prices of drug significantly vary across drugs. For example, unit price of one drug may cost to $ 0.5 dollar and unit price of another drug may cost $ 100, and it creates challenges in comparison of reasons for those prices and more particularly comparison of entities of pharma players responsible for the price variation. In an instance, PBM is termed as a pharma player and each PBM is termed as an entity. Grouping of the prices within a drug enables comparison of price of drugs based on a common scale such as low to very high and as grouping of prices is done for each drug, it enables comparison of reasons across drugs with huge variations in prices. Price of each transaction is labelled as any one of the group based on the price value and price ranges of each price group and each transaction is labelled as any one among the low, medium, high and very high. The process of grouping of prices enables to generate three dimensional view which is described in later sections.

Further, at step 206 of the method 200, the system 100 maps each of the of the plurality of predefined price groups of each of the plurality of drugs to at least one of a plurality of entities, and a frequency of each of the plurality of entities against each price group from among the plurality of predefined price groups for each of the plurality of drugs for the dynamic time period specific to each of the plurality of drugs is calculated. At this step, the system 100 maps each transaction with corresponding labelled price group with pharma players using one or more appropriate methods and frequency of each entity for each price group is calculated for each drug separately as per the FIG. 3A. As per the FIG. 3A, frequency of each entity is calculated for each price group formed and the procedure is repeated for each drug. In an instance, PBM is termed as a pharma player and each PBM is termed as an entity and same notation is followed for each pharma player. Overall frequency of each entity is calculated by aggregating frequency of corresponding entity across drugs, at step 208 of the method 200. The overall frequency for each price group is derived by considering all the drugs and using the standardized pricing groups. Thus, the overall frequency captures nature of pricing of each entity of each pharma player, across drugs. It is obvious for those skilled in the art that the patterns or frequency followed by two entities across price groups decide the relationship between the two entities. The table as in FIG. 3A has specific format of rows and columns to capture two kinds of interrelationship such as (i) the interrelationship between entities of pharma players for each drug, and (ii) the interrelationship between price groups and entities of pharma players for each drug. In addition, frequency of each entity for a price group is depending on the price range of the price group. In an instance, for the first drug, first cell in the Table in FIG. 3A displays the frequency of PBM_1 for low price group having the range as $2.1 to $2.8. If price point $ 2.8 is moved to adjacent price group due to many reasons such as refinement of price groups, etc., the range is reduced as $ 2.1 to $ 2.7, and the frequency of PBM_1 will reduce. Thus depending on the movement of price point from one group to adjacent group, the frequency of each entity for each price group changes, leading to changes in the two kinds of interrelationships.

Further, at step 210 of the method 200, a correspondence dimension matrix is estimated via the one or more hardware processors, based on the overall frequency of the plurality of entities for each of the plurality of predefined price groups, wherein the correspondence dimension matrix comprises a set of dimensions representing direction of each of the plurality of entities in relation to other entities among the plurality of entities. The processed data with price labels and corresponding overall frequency of entities of pharma players as per final rows in the FIG. 3A are applied with specific type of analysis as per standard analytical procedures. The overall frequency for each price group is derived by considering all the drugs and using the standardized pricing groups and thus it captures nature of pricing followed by each entity of pharma players across drugs. Final set of rows with overall frequency for predefined price groups against each entity of each pharma player is applied with correspondence analysis. In an embodiment, a correspondence analysis is used when variables are categorical in nature. The correspondence analysis is a multivariate technique that is especially powerful for finding patterns among combinations of variables in a large dataset. The correspondence analysis is applied using the final set of rows with overall frequency for predefined price groups against each entity of each pharma player as displayed in Fig 3A and the correspondence dimension matrix is generated. Final set of rows with overall frequency will have 4 rows in which each row belongs to the price group named as low, medium, high, and very high and each row with overall frequency captures nature of pricing by considering all drugs without any numerical price ranges. This set up enables to grade the level of pricing across drugs based on common scale such as low to very high. As per standard correspondence analysis, when 4 price groups with corresponding overall frequency are used as input, three dimensions are generated and variance contribution of each dimension and loadings of each entity under each dimension are provided by the correspondence analysis. If a dimension has more variance contribution, it indicates that some of the entities of the dimension are closer as much as possible. The dimensions are orthogonal to each other, and it ensures the deviation from other entities of other dimensions as much as possible.

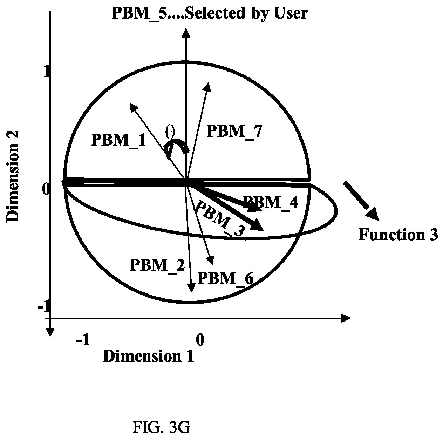

Further, at step 212 of the method 200, a magnitude of interrelationship between the plurality of entities is extracted, via the one or more hardware processors 102, using the set of dimensions in the correspondence dimension matrix. Further, at step 214 of the method 200, a pharmaceutical pricing guide is formed, via the one or more hardware processors, based on the magnitude of interrelationship between the plurality of entities. An example of the pharmaceutical pricing guide is depicted in FIG. 3G.

For example, Table. 1 shows sample correspondence dimension matrix having name of the entity (PBM1) of a pharma player (PBM) and loading value of each dimension for the corresponding entity.

Table. 1

| Pharma players | Dimension 1 | Dimension 2 | Dimension 3 |
|---|---|---|---|
| PBM_1 | 0.4 | 0.9 | 0.3 |
| PBM_2 | -0.3 | -0.9 | -0.4 |
| PBM_3 | -0.4 | -0.5 | 0.9 |
| PBM_4 | 0.2 | 0.6 | 0.7 |

(continued)

| Pharma players | Dimension 1 | Dimension 2 | Dimension 3 |
|---|---|---|---|
| PBM_5 | 0.8 | 0.6 | 0.25 |
| PBM_6 | -0.85 | -0.2 | -0.4 |
| PBM_7 | -0.95 | -0.3 | 0.2 |
| PBM_8 | 0.1 | 0.4 | 0.65 |
| Insurer_1 | 0.3 | 0.8 | 0.2 |
| Insurer_2 | -0.9 | -0.2 | -0.4 |
| Insurer_3 | -0.4 | -0.3 | 0.8 |
| Insurance Plan_1 | 0.35 | 0.85 | 0.25 |
| Insurance Plan_2 | -0.85 | -0.15 | -0.27 |
| Insurance Plan_3 | -0.5 | -0.17 | 0.85 |
| Brand_1 | 0.25 | 0.75 | 0.15 |
| Brand_2 | -0.95 | -0.05 | -0.2 |
| Brand_3 | -0.23 | -0.42 | 0.75 |
| ....... | | | |
| ....... | | | |

Each row of the correspondence dimension matrix indicates direction of an entity in relation to other entities. The correspondence dimension matrix includes values for each entity of a player in the pharmaceutical domain. The correspondence dimension matrix has coefficient or loading values for each entity of pharma player under each correspondence dimension. Correspondence dimensions are orthogonal to each other.

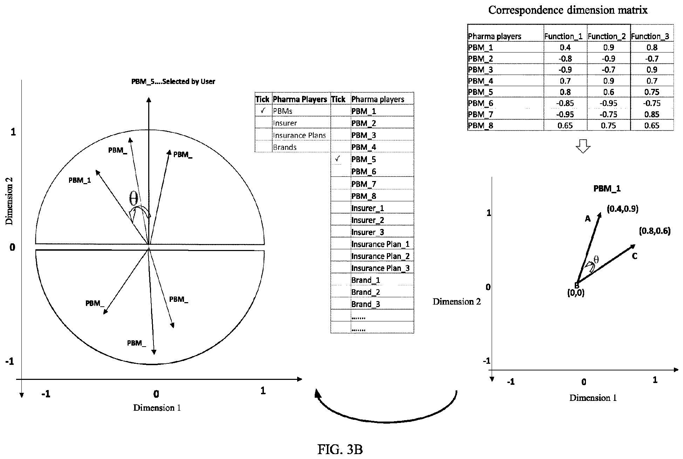

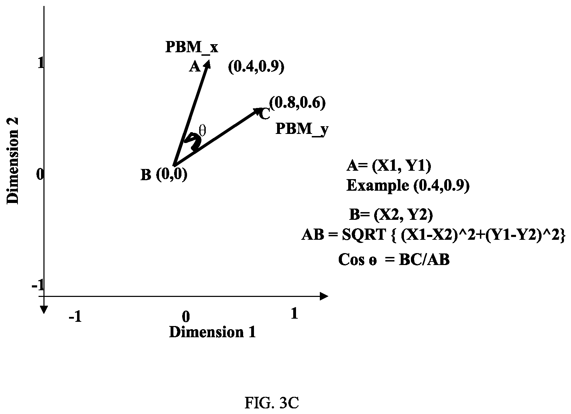

The system 100 may use any suitable approach for calculating angle between two entities of a player. In an embodiment, an approach used for calculating the angle is explained with reference to FIGS. 3B, 3C and Table 1.

As in FIGS. 3B and 3C, distance between centroid, B, noted by the coordinate (X1,Y1) (example - (0,0) ) and an entity, A, noted by the coordinate (X2,Y2) (example - (0.4,0.9) ) is calculated using equation (1).

$$Dist\ (AB) = SQRT\ \{\ (X1\text{-}X2)^2 + (Y1\text{-}Y2)^2\} \qquad ............... (1)$$

Where, SQRT indicates square root and (0.4, 0.9) are the loading value for an entity (PBM_1) for dimension 1 and 2 as per Table1.

Same way, distance between centroid (0,0) and another entity, C (example - (0.8,0.6) ) is calculated using the equation (1) and is denoted as Dist (BC). Further, value of Cos θ is calculated using relationship in Equation 2.

$$Cos\ \theta\ =\ Dist\ (BC) / Dist\ (AB)$$

$$Where\ (0.8,\ 0.6)\ are\ the\ loading\ value\ for\ another\ entity\ (PBM\_5)\ for$$

$$dimension\ 1\ and\ 2\ as\ per\ Table1. ............. (2)$$

The angle θ (theta) is calculated from cos e using standard trigonometric functions. The θ is the angle between two entities of a player (PBM) which indicates about how these two entities act with respect to pharmaceutical pricing. This process is repeated for similar combination of all entities of different players, and the corresponding angles are obtained. These values represent the magnitude of interrelationship between the plurality of entities. The angles calculated between two entities and across all entities are stored in the system 100 and they may be used as key inputs for different decision support systems in pharmaceutical domain. In one embodiment, it is used to form a pharmaceutical pricing guide which is used to view the nature of pricing strategies followed by different entities of pharma players. For two-dimensional configuration in any applications, top two correspondence dimensions and their loading values for each entity are considered to calculate the angle between entities and to explore behavior of each entity with other entity within a player and behavior of entities across players. Top two correspondence dimensions are selected based on magnitude of variance contribution of dimensions. For configuration of three-dimensional display in any applications, all the three correspondence dimensions are considered to calculate the angle between entities.

Further, the system 100 measures quality of the pharmaceutical pricing guide in relation to its usage, in light of one or more pre-defined criteria. For example, the criteria maybe that similar entities in terms of nature of pricing need to have a minimum angle between themselves as much as possible and different pharma entities in terms of nature of pricing need to have maximum angle between themselves as much as possible. Quality of pricing guide for each entity is obtained from (a) sum of variance contributions by all the dimensions of correspondence analysis and (b) maximum loading value for the entity in any of the dimensions of correspondence analysis in which the entity refers to the entity selected in the application. Sum of variance contributions by all the dimensions acts as first level target and maximum loading value of the entity in any of the dimension acts as second level target. Quality of pricing guide in terms of the first level target for each drug for each entity is measured from sum of variance contributions by the three dimensions. If a dimension has more variance contribution, it indicates that some of the entities are closer to each other as much as possible within the dimension. As dimensions are orthogonal to each other it ensures the deviation of the entities of a dimension from the entities of other dimensions as much as possible. Thus, variance contribution by a dimension acts as a measure to indicate closeness of certain entities of the dimension and sum of variance contribution by all the dimensions acts as a measure to indicate closeness of certain entities and deviations with other entities. Quality of pricing guide in terms of the second level target for each drug for each entity is measured from maximum possible loading in any one dimension. One entity needs to have maximum possible loading in any one dimension and minimum possible loading in other dimensions so that the length of arrow for the selected entity in the pricing guide is maximum. Thus, sum of variance contribution by the three dimensions and maximum possible loading for the entity selected in any one dimension indicates the quality of pricing guide.

Further, at step 216 of the method 200, the system 100 maximizes the measured quality of the pharmaceutical pricing guide for each of the plurality of entities, via the one or more hardware processors, in real time, wherein the measured quality is maximized by (i) self-adjusting of price point across the plurality of predefined price groups for each of the plurality of drugs, (ii) self-adjusting time period specific to each of the plurality of drugs, and by (iii) considering a selected entity from among the plurality of entities in real time and a frequency distribution of the selected entity across the plurality of predefined price groups. Maximizing the quality in the context of the embodiments disclosed herein indicates improving/increasing the quality of the pharmaceutical pricing guide. Maximizing the quality of the pricing guide for each drug for each entity is achieved by iterations. Border points for a price group are identified and ordered for each iteration and moved to nearest group one by one based on the order until the quality of the pricing guide is maximized. The procedure is repeated for each drug and each price group in which the quality of pricing guide is measured at every movement of price point by running correspondence analysis with updated price groups due to each movement of price point to adjacent price group. Adjacent price group is decided based on average of price groups.

Thus, depending on the movement of price point from one group to adjacent group for each drug, the frequency of each entity for each price group for each drug changes, leading to changes in the interrelationship between entities of pharma players. Movement of price point for each drug to adjacent price groups results in changes in the overall frequency of each entity for each price group which is used as input for correspondence analysis. Thus, it leads to dynamic input for correspondence analysis and changes in the correspondence dimension matrix. If the measured quality is not changing over consecutive iterations with movement of price point for a drug to adjacent price groups, then the iterations for the drug may be terminated and the procedure is shifted to next drug. Thus, the procedure is repeated for each drug one by one and until all drugs are tested for quality improvement. The system 100 finds an ideal price range for each price group and for each drug by iterative ways by movement of price points to adjacent price groups so that similar entities in terms of nature of pricing have minimum angle between themselves as much as possible and different entities in terms of nature of pricing have maximum angle between themselves as much as possible. It is ensured from maximization of sum of variance contributions by the three dimensions of correspondence analysis.

Once maximum variance is achieved it indicates that the first level target is achieved and it is maintained in the next level processing. Movement of price point across price group is again carried out in real time to attain maximum possible loading in any one of the dimensions and minimum loadings for the remaining dimensions for the entity selected by the user, by ensuring that the first level target of maximum variance is maintained. Maximum possible loading for the entity selected enables to get maximum possible length of the arrow for the selected entity in the pricing guide. In FIG. 3B, PBM 1, has the value as (0.4, 0.9). In one instance, movement of price point may fine tune the value as (0.3, 0.96) in which loading for the dimension 2 is improved from 0.9 to 0.96 and loading for the dimension 1 is decreased from 0.4 to 0.3, thus increasing the distance AB or length of arrow AB. Ideally one entity needs to have maximum possible loading in one dimension and minimum possible loading in the other dimensions so that the length of arrow for the selected entity in the pricing guide will be maximum. Once maximum possible loading for the entity selected is reached by iterations of price value movement across price groups, it indicates second level target is achieved.

By similar way the time period considered for each drug varies. The system 100 is configured to find ideal time period for each drug by iterative ways so that quality of pricing guide is maximized by ensuring similar entities in terms of nature of pricing have minimum angle between themselves as much as possible and different entities in terms of nature of pricing have maximum angle between themselves as much as possible and the entity selected has maximum possible loading in one dimension and minimum possible loading in the other dimensions so that the length of arrow for the selected entity in the pricing guide is maximum as much as possible.

In various embodiments, increasing the quality of the pharmaceutical pricing guide may be done over one or more iterations, till the measured quality has reached a saturation point. For example, if the measured quality is not changing over consecutive iterations of maximizing the quality, or if a measured improvement is below a threshold, then the step of maximizing the quality maybe terminated.

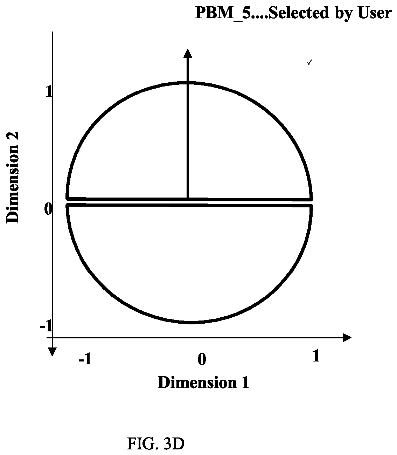

The pharmaceutical pricing guide, after maximizing the quality, maybe then used at step 218 of the method 200, to perform an application specific entity selection, via the one or more hardware processors. This is explained with reference to FIGS. 3C through 3H. Initially, coordinates of a base axis for the entity selected is fixed. For example, as in FIG. 3D, (0,0) and (0,1) are fixed as the base axis. The base axis acts as the axis for entity interested in or chosen by a user. The other lines are drawn based on the angle between the entity interested and other entities which were calculated and stored in a previous section.

For example when the user is interested to compare entities of PBM, the user has to select the entities of PBM in the check box as displayed in FIG 3B. When a user selects an entity within a player, the entity is fixed with the determined coordinates i.e. (0,0) and (0,1) as displayed in the FIG. 3D. The user may be a retail pharmacist or an analyst working in a retail pharmacy or a patient and the like.. Based on the selection of entity of a pharma player, the other entities are displayed as per the angle between the entity selected and the other entities, which were stored in the system.

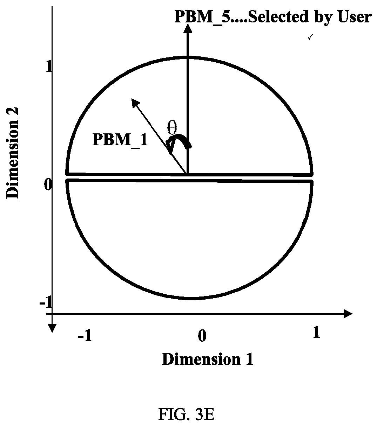

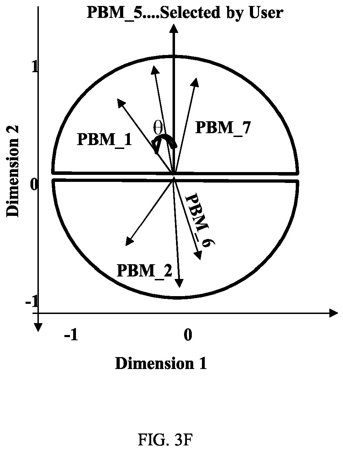

FIG 3E to FIG 3G have sample scenarios indicating different possibilities of relationship across entities of PBM. In an instance, as displayed in FIG 3E, when a retail pharmacist reviews PBM_5 who is the existing major partner, PBM_1 may have nature of pricing which is slightly deviating from PBM_5 and the deviating amount is reflected from the theta. It indicates that in an instance, if PBM_5 follows low pricing strategy across drugs, then PBM_1 also may follow low pricing strategy across drugs with slight deviation from PBM_5. Here, comparison of nature of pricing is done on overall basis by considering all the drugs based on a common scale such as low to very high which was described in earlier sections in which standardization of pricing across drugs was enabled through grouping of price points. As displayed in FIG 3F, PBM_2 may have entirely opposite behavior as compared to PBM_5, and PBM_2 may have slightly deviating behavior as compared to PBM_6. In an instance, as PBM_5 follows low pricing strategy, PBM_2 may follow high pricing strategy. PBM_6 also may follow high pricing strategy with slight deviation from PBM_2. As displayed in FIG. 3G, PBM_3 and PBM_4 may have opposite behavior for both PBM_5 and PBM_2. It indicates that in an instance, as PBM_5 follows low pricing strategy and PBM_2 follows high pricing strategy, PBM_3 and PBM_4 may follow medium pricing strategy.

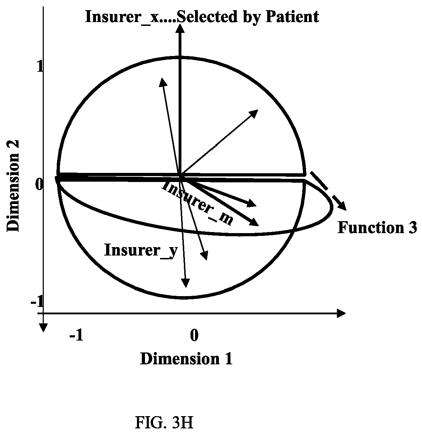

In another example, as displayed in FIG. 3H, when a patient reviews insurer_x who is the existing insurer for him/her, insurer_y may have entirely opposite behavior as compared to insurer_x. Insurer _m may have opposite behavior for both insurer_x and insurer_y. In another example, the user may be interested in reviewing entities across players. For example when the user is interested to compare PBM and insurance plan simultaneously, the user has to select both the options in the check box, and corresponding entities are displayed using the calculated angles across entities across dimensions.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

The embodiments of present disclosure herein address unresolved problem of pricing analysis in pharmaceutical domain. The embodiment, thus provides a mechanism of forming a pharmaceutical pricing guide based on a determined magnitude of interrelationship between a plurality of entities of a pharma player. Moreover, the embodiments herein further provide a mechanism of performing application specific entity selection based on the pharmaceutical pricing guide.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A processor implemented method (200), comprising:

   receiving (202), via one or more hardware processors, a set of information associated with each of a plurality of drugs for a predefined period of time, as input data;
   generating (204) a plurality of predefined price groups for each of the plurality of drugs, via the one or more hardware processors, wherein the plurality of predefined price groups for each of the plurality of drugs are generated by grouping the set of information in the input data at a transaction level for a dynamic time period specific to each of the plurality of drugs, wherein the input data contained in an external database is periodically updated and new data is added into the external database, and existing data is modified while non-useful data is deleted from the external database;
   mapping (206), via the one or more hardware processors, each of the of the plurality of predefined price groups of each of the plurality of drugs to at least one of a plurality of entities and calculating frequency of each of the plurality of entities against each price group from among the plurality of predefined price groups for each of the plurality of drugs for the dynamic time period specific to each of the plurality of drugs, wherein the dynamic time period refers to a particular time period that is opted by an user and information corresponding to the particular time period from the input data forms set of information that is grouped by the system;
   aggregating (208), via the one or more hardware processors, frequency of each of the plurality of entities for each of the plurality of predefined price groups for each of the plurality of drugs to derive an overall frequency for each of the plurality of entities for each of the plurality of predefined price groups;
   estimating (210), via the one or more hardware processors, a correspondence dimension matrix based on the overall frequency of the plurality of entities for each of the plurality of predefined price groups, wherein the correspondence dimension matrix comprises a set of dimensions representing direction of each of the plurality of entities in relation to other entities among the plurality of entities;
   extracting (212), via the one or more hardware processors, a magnitude of interrelationship between the plurality of entities using the set of dimensions in the correspondence dimension matrix;
   forming (214), via the one or more hardware processors, a pharmaceutical pricing guide based on the magnitude of interrelationship between the plurality of entities;

maximizing (216), via the one or more hardware processors, a measured quality of the pharmaceutical pricing guide for each of the plurality of entities in real time, wherein the measured quality is maximized by (i) self-adjusting of price point across the plurality of predefined price groups for each of the plurality of drugs, (ii) self-adjusting time period specific to each of the plurality of drugs, and by (iii) considering a selected entity from among the plurality of entities in real time and a frequency distribution of the selected entity across the plurality of predefined price groups, wherein maximizing the measured quality of the pharmaceutical pricing guide is achieved in a plurality of iterations, wherein in each of the plurality of iterations, (i) a plurality of border points for each of the plurality of predefined price groups for each of the plurality of drugs are identified and ordered and are moved to an adjacent price group one by one based on the order until the quality of the pharmaceutical pricing guide is maximized, and (ii) time periods specific to each of the plurality of drugs is changed until the quality of the pharmaceutical pricing guide is maximized, wherein the quality of pharmaceutical pricing guide is measured at every movement of the price point and at every change in time period specific to each of the plurality of drugs by running a correspondence analysis with an updated set of price group obtained due to each movement of the price point to the adjacent price group, wherein the adjacent price group is decided based on price group means, wherein movement of price point for each drug to adjacent price groups results in changes in the overall frequency of each entity for each price group and the changes in overall frequency leads to dynamic input for correspondence analysis and changes in a correspondence matrix if a measured quality is not changing over consecutive iterations with movement of the price point for a drug to adjacent price groups; and

performing (218), via the one or more hardware processors, an application specific entity selection using the pharmaceutical pricing guide.

2. The method as claimed in claim 1, wherein the input data comprises one or more of a) drug related information, b) pharmacy related information, and c) insurance related information.

3. The method as claimed in claim 1, wherein the number of price groups is predefined and fixed as four to enable to generate one or more of two-dimensional or three-dimensional views.

4. The method as claimed in claim 1, wherein the correspondence dimension matrix is estimated by applying a correspondence analysis using the overall frequency for the plurality of entities for each of the plurality of pre-defined price groups.

5. The method as claimed in claim 1, wherein for each of the plurality of entities selected for the application, the measured quality of pharmaceutical pricing guide is obtained from (a) sum of variance contributions by all dimensions of the correspondence analysis, and (b) maximum loading value for the entity in any one of the dimensions of correspondence analysis.

6. The method as claimed in claim 1, wherein the magnitude of interrelationship between the plurality of entities is extracted by applying a plurality of trigonometric functions on the set of dimensions, wherein the plurality of trigonometric functions comprises distance between a centroid with coordinate $(X_m, Y_m)$ and an entity with coordinate $(X_n, Y_n)$.

7. A system (100), comprising:

one or more hardware processors (102);
a communication interface (112); and
a memory (104) storing a plurality of instructions, wherein the plurality of instructions cause the one or more hardware processors to:

receive a set of information associated with each of a plurality of drugs for a predefined period of time, as input data, wherein the input data contained in an external database is periodically updated and new data is added into the external database, and existing data is modified while non-useful data is deleted from the external database;
generate a plurality of predefined price groups for each of the plurality of drugs, wherein the plurality of predefined price groups for each of the plurality of drugs are generated by grouping the set of information in the input data at a transaction level for a dynamic time period specific to each of the plurality of drugs;
map each of the of the plurality of predefined price groups of each of the plurality of drugs to at least one of a plurality of entities and calculating frequency of each of the plurality of entities against each price group from among the plurality of predefined price groups for each of the plurality of drugs for the dynamic time period specific to each of the plurality of drugs, wherein the dynamic time period refers to a particular time period that is opted by an user and information corresponding to the particular time period from the input data forms set of information that is grouped by the system;

aggregate frequency of each of the plurality of entities for each of the plurality of predefined price groups for each of the plurality of drugs to derive an overall frequency for each of the plurality of entities for each of the plurality of predefined price groups;

estimate a correspondence dimension matrix based on the overall frequency of the plurality of entities for each of the plurality of predefined price groups, wherein the correspondence dimension matrix comprises a set of dimensions representing direction of each of the plurality of entities in relation to other entities among the plurality of entities;

extract a magnitude of interrelationship between the plurality of entities using the set of dimensions in the correspondence dimension matrix;

form a pharmaceutical pricing guide based on the magnitude of interrelationship between the plurality of entities;

maximize a measured quality of the pharmaceutical pricing guide for each of the plurality of entities in real time, wherein the measured quality is maximized by (i) self-adjusting of price point across the plurality of predefined price groups for each of the plurality of drugs for each of the plurality of entities in real time, (ii) self-adjusting time period specific to each of the plurality of drugs, and by (iii) considering a selected entity from among the plurality of entities in real time and a frequency distribution of the selected entity across the plurality of predefined price groups, wherein maximizing the measured quality of the pharmaceutical pricing guide is achieved in a plurality of iterations, wherein in each of the plurality of iterations, (i) a plurality of border points for each of the plurality of predefined price groups for each of the plurality of drugs are identified and ordered and are moved to an adjacent price group one by one based on the order until the quality of the pharmaceutical pricing guide is maximized, and (ii) time periods specific to each of the plurality of drugs is changed until the quality of the pharmaceutical pricing guide is maximized, wherein the quality of pharmaceutical pricing guide is measured at every movement of the price point and at every change in time period specific to each of the plurality of drugs by running a correspondence analysis with an updated set of price group obtained due to each movement of the price point to the adjacent price group, wherein the adjacent price group is decided based on price group means, wherein movement of price point for each drug to adjacent price groups results in changes in the overall frequency of each entity for each price group and the changes in overall frequency leads to dynamic input for correspondence analysis and changes in a correspondence matrix if a measured quality is not changing over consecutive iterations with movement of the price point for a drug to adjacent price groups; and

perform an application specific entity selection using the pharmaceutical pricing guide.

8.  The system as claimed in claim 7, wherein the one or more hardware processors are configured to receive one or more of a) drug related information, b) pharmacy related information, and c) insurance related information, as the input data.

9.  The system as claimed in claim 7, wherein the one or more hardware processors are configured to predefine and fix the number of price groups as four to enable to generate one or more of two-dimensional or three-dimensional views.

10. The system as claimed in claim 7, wherein the one or more hardware processors are configured to estimate the correspondence dimension matrix by applying a correspondence analysis using the overall frequency for the plurality of entities for each of the plurality of pre-defined price groups.

11. The system as claimed in claim 7, wherein the one or more hardware processors are configured to obtain the measured quality of pharmaceutical pricing guide for each of the plurality of entities from (a) sum of variance contributions by all dimensions of the correspondence analysis, and (b) maximum loading value for the entity in any one of the dimensions of correspondence analysis.

12. The system as claimed in claim 7, wherein the one or more hardware processors are configured to extract the magnitude of interrelationship between the plurality of entities by applying a plurality of trigonometric functions on the set of dimensions, wherein the plurality of trigonometric functions comprises distance between a centroid with coordinate $(X_m, Y_m)$ and an entity with coordinate $(X_n, Y_n)$.

13. One or more non-transitory machine-readable information storage mediums comprising one or more instructions which when executed by one or more hardware processors cause:

receiving a set of information associated with each of a plurality of drugs for a predefined period of time, as input data, wherein the input data contained in an external database is periodically updated and new data is added into the external database, and existing data is modified while non-useful data is deleted from the external database;

generating a plurality of predefined price groups for each of the plurality of drugs, wherein the plurality of predefined price groups for each of the plurality of drugs are generated by grouping the set of information in the input data at a transaction level for a dynamic time period specific to each of the plurality of drugs;

mapping each of the of the plurality of predefined price groups of each of the plurality of drugs to at least one of a plurality of entities and calculating frequency of each of the plurality of entities against each price group from among the plurality of predefined price groups for each of the plurality of drugs for the dynamic time period specific to each of the plurality of drugs, wherein the dynamic time period refers to a particular time period that is opted by an user and information corresponding to the particular time period from the input data forms set of information that is grouped by the system;

aggregating frequency of each of the plurality of entities for each of the plurality of predefined price groups for each of the plurality of drugs to derive an overall frequency for each of the plurality of entities for each of the plurality of predefined price groups;

estimating a correspondence dimension matrix based on the overall frequency of the plurality of entities for each of the plurality of predefined price groups, wherein the correspondence dimension matrix comprises a set of dimensions representing direction of each of the plurality of entities in relation to other entities among the plurality of entities;

extracting a magnitude of interrelationship between the plurality of entities using the set of dimensions in the correspondence dimension matrix;

forming a pharmaceutical pricing guide based on the magnitude of interrelationship between the plurality of entities;

maximizing a measured quality of the pharmaceutical pricing guide for each of the plurality of entities in real time, wherein the measured quality is maximized by (i) self-adjusting of price point across the plurality of predefined price groups for each of the plurality of drugs, (ii) self-adjusting time period specific to each of the plurality of drugs, and by (iii) considering a selected entity from among the plurality of entities in real time and a frequency distribution of the selected entity across the plurality of predefined price groups, wherein maximizing the measured quality of the pharmaceutical pricing guide is achieved in a plurality of iterations, wherein in each of the plurality of iterations, (i) a plurality of border points for each of the plurality of predefined price groups for each of the plurality of drugs are identified and ordered and are moved to an adjacent price group one by one based on the order until the quality of the pharmaceutical pricing guide is maximized, and (ii) time periods specific to each of the plurality of drugs is changed until the quality of the pharmaceutical pricing guide is maximized, wherein the quality of pharmaceutical pricing guide is measured at every movement of the price point and at every change in time period specific to each of the plurality of drugs by running a correspondence analysis with an updated set of price group obtained due to each movement of the price point to the adjacent price group, wherein the adjacent price group is decided based on price group means, wherein movement of price point for each drug to adjacent price groups results in changes in the overall frequency of each entity for each price group and the changes in overall frequency leads to dynamic input for correspondence analysis and changes in a correspondence matrix if a measured quality is not changing over consecutive iterations with movement of the price point for a drug to adjacent price groups; and

performing an application specific entity selection using the pharmaceutical pricing guide.

## Patentansprüche

1. Prozessorimplementiertes Verfahren (200), umfassend:

Empfangen (202), über einen oder mehrere Hardwareprozessoren, eines Satzes von Informationen, die mit jedem einer Mehrzahl von Medikamenten assoziiert sind, für einen vordefinierten Zeitraum als Eingabe-Daten;

Erzeugen (204) einer Mehrzahl von vordefinierten Preisgruppen für jedes der Mehrzahl von Medikamenten, über den einen oder die mehreren Hardwareprozessoren, wobei die Mehrzahl von vordefinierten Preisgruppen für jedes der Mehrzahl von Medikamenten durch Gruppieren des Satzes von Informationen in den Eingabe-Daten auf einer Transaktionsebene für einen dynamischen Zeitraum, der für jedes der Mehrzahl von Medikamenten spezifisch ist, erzeugt wird, wobei die Eingabe-Daten, die in einer externen Datenbank enthalten sind, periodisch aktualisiert werden und neue Daten in die externe Datenbank hinzugefügt werden und vorhandene Daten modifiziert werden, während nicht nützliche Daten aus der externen Datenbank gelöscht werden;

Abbilden (206), über den einen oder die mehreren Hardwareprozessoren, jeder der Mehrzahl von vordefinierten Preisgruppen von jedem der Mehrzahl von Medikamenten auf mindestens eine einer Mehrzahl von Entitäten und Berechnen der Häufigkeit jeder der Mehrzahl von Entitäten gegen jede Preisgruppe aus der Mehrzahl von vordefinierten Preisgruppen für jedes der Mehrzahl von Medikamenten für den dynamischen Zeitraum, der für jedes der Mehrzahl von Medikamenten spezifisch ist, wobei sich der dynamische Zeitraum auf einen bestimmten Zeitraum bezieht, der von einem Benutzer gewählt wird, und Informationen, die dem bestimmten Zeitraum aus den Eingabe-Daten entsprechen, einen Satz von Informationen bilden, der von dem System gruppiert wird;

Aggregieren (208), über den einen oder die mehreren Hardwareprozessoren, der Häufigkeit jeder der Mehrzahl von Entitäten für jede der Mehrzahl von vordefinierten Preisgruppen für jedes der Mehrzahl von Medikamenten, um eine Gesamthäufigkeit für jede der Mehrzahl von Entitäten für jede der Mehrzahl von vordefinierten Preisgruppen abzuleiten;

Schätzen (210), über den einen oder die mehreren Hardwareprozessoren, einer Entsprechungsdimensions-Matrix basierend auf der Gesamthäufigkeit der Mehrzahl von Entitäten für jede der Mehrzahl von vordefinierten Preisgruppen, wobei die Entsprechungsdimensions-Matrix einen Satz von Dimensionen umfasst, die die Richtung jeder der Mehrzahl von Entitäten in Bezug auf andere Entitäten aus der Mehrzahl von Entitäten darstellen;

Extrahieren (212), über den einen oder die mehreren Hardwareprozessoren, einer Größe der Wechselbeziehung zwischen der Mehrzahl von Entitäten unter Verwendung des Satzes von Dimensionen in der Entsprechungsdimensions-Matrix;

Bilden (214), über den einen oder die mehreren Hardwareprozessoren, eines pharmazeutischen Preisleitfadens basierend auf der Größe der Wechselbeziehung zwischen der Mehrzahl von Entitäten;

Maximieren (216), über den einen oder die mehreren Hardwareprozessoren, einer gemessenen Qualität des pharmazeutischen Preisleitfadens für jede der Mehrzahl von Entitäten in Echtzeit, wobei die gemessene Qualität maximiert wird durch (i) Selbstanpassung des Preispunkts über die Mehrzahl von vordefinierten Preisgruppen für jedes der Mehrzahl von Medikamenten, (ii) Selbstanpassung der Zeitdauer, die für jedes der Mehrzahl von Medikamenten spezifisch ist, und durch (iii) Berücksichtigen einer ausgewählten Entität aus der Mehrzahl von Entitäten in Echtzeit und einer Häufigkeitsverteilung der ausgewählten Entität über die Mehrzahl von vordefinierten Preisgruppen, wobei das Maximieren der gemessenen Qualität des pharmazeutischen Preisleitfadens in einer Mehrzahl von Iterationen erreicht wird, wobei in jeder der Mehrzahl von Iterationen (i) eine Mehrzahl von Grenzpunkten für jede der Mehrzahl von vordefinierten Preisgruppen für jedes der Mehrzahl von Medikamenten identifiziert und geordnet werden und zu einer benachbarten Preisgruppe nacheinander basierend auf der Reihenfolge bewegt werden, bis die Qualität des pharmazeutischen Preisleitfadens maximiert ist, und (ii) Zeitdauern, die für jedes der Mehrzahl von Medikamenten spezifisch sind, geändert werden, bis die Qualität des pharmazeutischen Preisleitfadens maximiert ist, wobei die Qualität des pharmazeutischen Preisleitfadens bei jeder Bewegung des Preispunkts und bei jeder Änderung der Zeitdauer, die für jedes der Mehrzahl von Medikamenten spezifisch ist, durch Ausführen einer Entsprechungsanalyse mit einem aktualisierten Satz von Preisgruppen, die aufgrund jeder Bewegung des Preispunkts zu der benachbarten Preisgruppe erhalten werden, gemessen wird, wobei die benachbarte Preisgruppe basierend auf Preisgruppenmitteln entschieden wird, wobei die Bewegung des Preispunkts für jedes Medikament zu benachbarten Preisgruppen zu Änderungen der Gesamthäufigkeit jeder Entität für jede Preisgruppe führt und die Änderungen der Gesamthäufigkeit zu einer dynamischen Eingabe für die Entsprechungsanalyse und Änderungen in einer Entsprechungsmatrix führen, wenn sich eine gemessene Qualität über aufeinanderfolgende Iterationen mit Bewegung des Preispunkts für ein Medikament zu benachbarten Preisgruppen nicht ändert; und

Durchführen (218), über den einen oder die mehreren Hardwareprozessoren, einer anwendungsspezifischen Entitätsauswahl unter Verwendung des pharmazeutischen Preisleitfadens.

2. Verfahren nach Anspruch 1, wobei die Eingabe-Daten eines oder mehrere von a) medikamentenbezogenen Informationen, b) apothekenbezogenen Informationen und c) versicherungsbezogenen Informationen umfassen.

3. Verfahren nach Anspruch 1, wobei die Anzahl von Preisgruppen vordefiniert und auf vier festgelegt ist, um zu ermöglichen, eine oder mehrere von zweidimensionalen oder dreidimensionalen Ansichten zu erzeugen.

4. Verfahren nach Anspruch 1, wobei die Entsprechungsdimensions-Matrix durch Anwenden einer Entsprechungsanalyse unter Verwendung der Gesamthäufigkeit für die Mehrzahl von Entitäten für jede der Mehrzahl von vordefinierten Preisgruppen geschätzt wird.

5. Verfahren nach Anspruch 1, wobei für jede der Mehrzahl von Entitäten, die für die Anwendung ausgewählt sind, die gemessene Qualität des pharmazeutischen Preisleitfadens aus (a) der Summe von Varianzbeiträgen durch alle Dimensionen der Entsprechungsanalyse und (b) dem maximalen Belastungswert für die Entität in einer der Dimensionen der Entsprechungsanalyse erhalten wird.

6. Verfahren nach Anspruch 1, wobei die Größe der Wechselbeziehung zwischen der Mehrzahl von Entitäten durch Anwenden einer Mehrzahl von trigonometrischen Funktionen auf den Satz von Dimensionen extrahiert wird, wobei die Mehrzahl von trigonometrischen Funktionen den Abstand zwischen einem Schwerpunkt mit Koordinate $(X_m, Y_m)$ und einer Entität mit Koordinate $(X_n, Y_n)$ umfasst.

7. System (100), umfassend:

einen oder mehrere Hardwareprozessoren (102);
eine Kommunikationsschnittstelle (112); und
einen Speicher (104), der eine Mehrzahl von Anweisungen speichert, wobei die Mehrzahl von Anweisungen den einen oder die mehreren Hardwareprozessoren zu Folgendem veranlasst:

Empfangen eines Satzes von Informationen, die mit jedem einer Mehrzahl von Medikamenten assoziiert sind, für einen vordefinierten Zeitraum als Eingabe-Daten, wobei die Eingabe-Daten, die in einer externen Datenbank enthalten sind, periodisch aktualisiert werden und neue Daten in die externe Datenbank hinzugefügt werden und vorhandene Daten modifiziert werden, während nicht nützliche Daten aus der externen Datenbank gelöscht werden;
Erzeugen einer Mehrzahl von vordefinierten Preisgruppen für jedes der Mehrzahl von Medikamenten, wobei die Mehrzahl von vordefinierten Preisgruppen für jedes der Mehrzahl von Medikamenten durch Gruppieren des Satzes von Informationen in den Eingabe-Daten auf einer Transaktionsebene für einen dynamischen Zeitraum, der für jedes der Mehrzahl von Medikamenten spezifisch ist, erzeugt wird;
Abbilden jeder der Mehrzahl von vordefinierten Preisgruppen von jedem der Mehrzahl von Medikamenten auf mindestens eine einer Mehrzahl von Entitäten und Berechnen der Häufigkeit jeder der Mehrzahl von Entitäten gegen jede Preisgruppe aus der Mehrzahl von vordefinierten Preisgruppen für jedes der Mehrzahl von Medikamenten für den dynamischen Zeitraum, der für jedes der Mehrzahl von Medikamenten spezifisch ist, wobei sich der dynamische Zeitraum auf einen bestimmten Zeitraum bezieht, der von einem Benutzer gewählt wird, und Informationen, die dem bestimmten Zeitraum aus den Eingabe-Daten entsprechen, einen Satz von Informationen bilden, der von dem System gruppiert wird;
Aggregieren der Häufigkeit jeder der Mehrzahl von Entitäten für jede der Mehrzahl von vordefinierten Preisgruppen für jedes der Mehrzahl von Medikamenten, um eine Gesamthäufigkeit für jede der Mehrzahl von Entitäten für jede der Mehrzahl von vordefinierten Preisgruppen abzuleiten;
Schätzen einer Entsprechungsdimensions-Matrix basierend auf der Gesamthäufigkeit der Mehrzahl von Entitäten für jede der Mehrzahl von vordefinierten Preisgruppen, wobei die Entsprechungsdimensions-Matrix einen Satz von Dimensionen umfasst, die die Richtung jeder der Mehrzahl von Entitäten in Bezug auf andere Entitäten aus der Mehrzahl von Entitäten darstellen;
Extrahieren einer Größe der Wechselbeziehung zwischen der Mehrzahl von Entitäten unter Verwendung des Satzes von Dimensionen in der Entsprechungsdimensions-Matrix;
Bilden eines pharmazeutischen Preisleitfadens basierend auf der Größe der Wechselbeziehung zwischen der Mehrzahl von Entitäten;
Maximieren einer gemessenen Qualität des pharmazeutischen Preisleitfadens für jede der Mehrzahl von Entitäten in Echtzeit, wobei die gemessene Qualität maximiert wird durch (i) Selbstanpassung des Preispunkts über die Mehrzahl von vordefinierten Preisgruppen für jedes der Mehrzahl von Medikamenten für jede der Mehrzahl von Entitäten in Echtzeit, (ii) Selbstanpassung eines Zeitraums, der für jedes der Mehrzahl von Medikamenten spezifisch ist, und durch (iii) Berücksichtigen einer ausgewählten Entität aus der Mehrzahl von Entitäten in Echtzeit und einer Häufigkeitsverteilung der ausgewählten Entität über die Mehrzahl von vordefinierten Preisgruppen, wobei das Maximieren der gemessenen Qualität des pharmazeutischen Preisleitfadens in einer Mehrzahl von Iterationen erreicht wird, wobei in jeder der Mehrzahl von Iterationen (i) eine Mehrzahl von Grenzpunkten für jede der Mehrzahl von vordefinierten Preisgruppen für jedes der Mehrzahl von Medikamenten identifiziert und geordnet werden und basierend auf der Reihenfolge nacheinander zu einer benachbarten Preisgruppe bewegt werden, bis die Qualität des pharmazeutischen Preisleitfadens maximiert ist, und (ii) Zeiträume, die für jedes der Mehrzahl von Medikamenten spezifisch sind, geändert werden, bis die Qualität des pharmazeutischen Preisleitfadens maximiert ist, wobei die Qualität des pharmazeutischen Preisleitfadens bei jeder Bewegung des Preispunkts und bei jeder Änderung des Zeitraums, der für jedes der Mehrzahl von Medikamenten spezifisch ist, durch Ausführen einer Entsprechungsanalyse mit einem aktualisierten Satz von Preisgruppen, die aufgrund jeder Bewegung des Preispunkts zu der benachbarten Preisgruppe erhalten werden, gemessen wird, wobei die benachbarte Preisgruppe basierend auf Preisgruppenmitteln entschieden wird, wobei die Bewegung des Preispunkts für jedes Medikament zu benachbarten Preisgruppen zu Änderungen der Gesamthäufigkeit jeder Entität für jede Preisgruppe führt und die Änderungen der Gesamthäufigkeit zu einer dynamischen Eingabe für die Entsprechungsanalyse und Änderungen einer Entsprechungsmatrix führen, wenn sich eine gemessene Qualität über aufeinanderfolgende Iterationen mit Bewegung des Preispunkts für ein Medikament zu benachbarten Preisgruppen nicht ändert; und

Durchführen einer anwendungsspezifischen Entitätsauswahl unter Verwendung des pharmazeutischen Preisleitfadens.

8. System nach Anspruch 7, wobei der eine oder die mehreren Hardwareprozessoren konfiguriert sind, um eines oder mehrere von a) medikamentenbezogenen Informationen, b) apothekenbezogenen Informationen und c) versicherungsbezogenen Informationen als die Eingabe-Daten zu empfangen.

9. System nach Anspruch 7, wobei der eine oder die mehreren Hardwareprozessoren konfiguriert sind, um die Anzahl von Preisgruppen als vier vorzudefinieren und festzulegen, um zu ermöglichen, eine oder mehrere von zweidimensionalen oder dreidimensionalen Ansichten zu erzeugen.

10. System nach Anspruch 7, wobei der eine oder die mehreren Hardwareprozessoren konfiguriert sind, um die Entsprechungsdimensions-Matrix durch Anwenden einer Entsprechungsanalyse unter Verwendung der Gesamthäufigkeit für die Mehrzahl von Entitäten für jede der Mehrzahl von vordefinierten Preisgruppen zu schätzen.

11. System nach Anspruch 7, wobei der eine oder die mehreren Hardwareprozessoren konfiguriert sind, um die gemessene Qualität des pharmazeutischen Preisleitfadens für jede der Mehrzahl von Entitäten aus (a) der Summe von Varianzbeiträgen durch alle Dimensionen der Entsprechungsanalyse und (b) dem maximalen Belastungswert für die Entität in einer der Dimensionen der Entsprechungsanalyse zu erhalten.

12. System nach Anspruch 7, wobei der eine oder die mehreren Hardwareprozessoren konfiguriert sind, um die Größe der Wechselbeziehung zwischen der Mehrzahl von Entitäten durch Anwenden einer Mehrzahl von trigonometrischen Funktionen auf den Satz von Dimensionen zu extrahieren, wobei die Mehrzahl von trigonometrischen Funktionen den Abstand zwischen einem Schwerpunkt mit Koordinate $(X_m, Y_m)$ und einer Entität mit Koordinate $(X_n, Y_n)$ umfasst.

13. Ein oder mehrere nichtflüchtige maschinenlesbare Informationsspeichermedien, umfassend eine oder mehrere Anweisungen, die, wenn sie von einem oder mehreren Hardwareprozessoren ausgeführt werden, Folgendes veranlassen:

Empfangen eines Satzes von Informationen, die mit jedem einer Mehrzahl von Medikamenten assoziiert sind, für einen vordefinierten Zeitraum als Eingabe-Daten, wobei die Eingabe-Daten, die in einer externen Datenbank enthalten sind, periodisch aktualisiert werden und neue Daten in die externe Datenbank hinzugefügt werden und vorhandene Daten modifiziert werden, während nicht nützliche Daten aus der externen Datenbank gelöscht werden; Erzeugen einer Mehrzahl von vordefinierten Preisgruppen für jedes der Mehrzahl von Medikamenten, wobei die Mehrzahl von vordefinierten Preisgruppen für jedes der Mehrzahl von Medikamenten durch Gruppieren des Satzes von Informationen in den Eingabe-Daten auf einer Transaktionsebene für einen dynamischen Zeitraum, der für jedes der Mehrzahl von Medikamenten spezifisch ist, erzeugt wird;

Abbilden jeder der Mehrzahl von vordefinierten Preisgruppen von jedem der Mehrzahl von Medikamenten auf mindestens eine einer Mehrzahl von Entitäten und Berechnen der Häufigkeit jeder der Mehrzahl von Entitäten gegen jede Preisgruppe aus der Mehrzahl von vordefinierten Preisgruppen für jedes der Mehrzahl von Medikamenten für den dynamischen Zeitraum, der für jedes der Mehrzahl von Medikamenten spezifisch ist, wobei sich der dynamische Zeitraum auf einen bestimmten Zeitraum bezieht, der von einem Benutzer gewählt wird, und Informationen, die dem bestimmten Zeitraum aus den Eingabe-Daten entsprechen, einen Satz von Informationen bilden, der von dem System gruppiert wird;

Aggregieren der Häufigkeit jeder der Mehrzahl von Entitäten für jede der Mehrzahl von vordefinierten Preisgruppen für jedes der Mehrzahl von Medikamenten, um eine Gesamthäufigkeit für jede der Mehrzahl von Entitäten für jede der Mehrzahl von vordefinierten Preisgruppen abzuleiten;

Schätzen einer Entsprechungsdimensions-Matrix basierend auf der Gesamthäufigkeit der Mehrzahl von Entitäten für jede der Mehrzahl von vordefinierten Preisgruppen, wobei die Entsprechungsdimensions-Matrix einen Satz von Dimensionen umfasst, die die Richtung jeder der Mehrzahl von Entitäten in Bezug auf andere Entitäten aus der Mehrzahl von Entitäten darstellen;

Extrahieren einer Größe der Wechselbeziehung zwischen der Mehrzahl von Entitäten unter Verwendung des Satzes von Dimensionen in der Entsprechungsdimensions-Matrix;

Bilden eines pharmazeutischen Preisleitfadens basierend auf der Größe der Wechselbeziehung zwischen der Mehrzahl von Entitäten;

Maximieren einer gemessenen Qualität des pharmazeutischen Preisleitfadens für jede der Mehrzahl von Entitäten in Echtzeit, wobei die gemessene Qualität maximiert wird durch (i) Selbstanpassung des Preispunkts über die Mehrzahl von vordefinierten Preisgruppen für jedes der Mehrzahl von Medikamenten, (ii) Selbstanpassung des Zeitraums, der für jedes der Mehrzahl von Medikamenten spezifisch ist, und durch (iii) Berücksichtigen einer ausgewählten Entität aus der Mehrzahl von Entitäten in Echtzeit und einer Häufigkeitsverteilung der ausgewählten Entität über die Mehrzahl von vordefinierten Preisgruppen, wobei das Maximieren der gemessenen Qualität des pharmazeutischen Preisleitfadens in einer Mehrzahl von Iterationen erreicht wird, wobei in jeder der Mehrzahl von Iterationen (i) eine Mehrzahl von Grenzpunkten für jede der Mehrzahl von vordefinierten Preisgruppen für jedes der Mehrzahl von Medikamenten identifiziert und geordnet werden und nacheinander basierend auf der Reihenfolge zu einer benachbarten Preisgruppe bewegt werden, bis die Qualität des pharmazeutischen Preisleitfadens maximiert ist, und (ii) Zeiträume, die für jedes der Mehrzahl von Medikamenten spezifisch sind, geändert werden, bis die Qualität des pharmazeutischen Preisleitfadens maximiert ist, wobei die Qualität des pharmazeutischen Preisleitfadens bei jeder Bewegung des Preispunkts und bei jeder Änderung des Zeitraums, der für jedes der Mehrzahl von Medikamenten spezifisch ist, gemessen wird, indem eine Entsprechungsanalyse mit einem aktualisierten Satz von Preisgruppen durchgeführt wird, der aufgrund jeder Bewegung des Preispunkts zu der benachbarten Preisgruppe erhalten wird, wobei die benachbarte Preisgruppe basierend auf Preisgruppenmitteln entschieden wird, wobei die Bewegung des Preispunkts für jedes Medikament zu benachbarten Preisgruppen zu Änderungen der Gesamthäufigkeit jeder Entität für jede Preisgruppe führt und die Änderungen der Gesamthäufigkeit zu einer dynamischen Eingabe für die Entsprechungsanalyse und Änderungen in einer Entsprechungsmatrix führen, wenn sich eine gemessene Qualität über aufeinanderfolgende Iterationen mit Bewegung des Preispunkts für ein Medikament zu benachbarten Preisgruppen nicht ändert; und

Durchführen einer anwendungsspezifischen Entitätsauswahl unter Verwendung des pharmazeutischen Preisleitfadens.

## Revendications

1. Procédé mis en œuvre par processeur (200), comprenant :

la réception (202), via un ou plusieurs processeurs matériels, d'un ensemble d'informations associées à chacun d'une pluralité de médicaments pendant une période de temps prédéfinie, en tant que données d'entrée ;

la génération (204) d'une pluralité de groupes de prix prédéfinis pour chacun de la pluralité de médicaments, via les un ou plusieurs processeurs matériels, dans lequel la pluralité de groupes de prix prédéfinis pour chacun de la pluralité de médicaments sont générés en regroupant l'ensemble d'informations dans les données d'entrée à un niveau de transaction pendant une période de temps dynamique spécifique à chacun de la pluralité de médicaments, dans lequel les données d'entrée contenues dans une base de données externe sont périodiquement mises à jour et de nouvelles données sont ajoutées dans la base de données externe, et des données existantes sont modifiées tandis que des données non utiles sont supprimées de la base de données externe ;

la mise en correspondance (206), via les un ou plusieurs processeurs matériels, de chacun de la pluralité de groupes de prix prédéfinis de chacun de la pluralité de médicaments avec au moins l'une d'une pluralité d'entités et le calcul de la fréquence de chacune de la pluralité d'entités par rapport à chaque groupe de prix parmi la pluralité de groupes de prix prédéfinis pour chacun de la pluralité de médicaments pendant la période de temps dynamique spécifique à chacun de la pluralité de médicaments, dans lequel la période de temps dynamique fait référence à une période de temps particulière qui est choisie par un utilisateur et des informations correspondant à la période de temps particulière à partir des données d'entrée forment un ensemble d'informations qui est regroupé par le système ;

l'agrégation (208), via les un ou plusieurs processeurs matériels, de la fréquence de chacune de la pluralité d'entités pour chacun de la pluralité de groupes de prix prédéfinis pour chacun de la pluralité de médicaments pour dériver une fréquence globale pour chacune de la pluralité d'entités pour chacun de la pluralité de groupes de prix prédéfinis ;

l'estimation (210), via les un ou plusieurs processeurs matériels, d'une matrice de dimensions de correspondance sur la base de la fréquence globale de la pluralité d'entités pour chacun de la pluralité de groupes de prix prédéfinis, dans lequel la matrice de dimensions de correspondance comprend un ensemble de dimensions représentant la direction de chacune de la pluralité d'entités par rapport à d'autres entités parmi la pluralité d'entités ;

l'extraction (212), via les un ou plusieurs processeurs matériels, d'une grandeur d'interrelation entre la pluralité d'entités en utilisant l'ensemble de dimensions dans la matrice de dimensions de correspondance ;

la formation (214), via les un ou plusieurs processeurs matériels, d'un guide de prix pharmaceutique sur la base de la grandeur d'interrelation entre la pluralité d'entités ;

la maximisation (216), via les un ou plusieurs processeurs matériels, d'une qualité mesurée du guide de prix pharmaceutique pour chacune de la pluralité d'entités en temps réel, dans lequel la qualité mesurée est maximisée par (i) l'auto-ajustement d'un point de prix à travers la pluralité de groupes de prix prédéfinis pour chacun de la pluralité de médicaments, (ii) l'auto-ajustement d'une période de temps spécifique à chacun de la pluralité de médicaments, et par (iii) la prise en compte d'une entité sélectionnée parmi la pluralité d'entités en temps réel et d'une distribution de fréquence de l'entité sélectionnée à travers la pluralité de groupes de prix prédéfinis, dans lequel la maximisation de la qualité mesurée du guide de prix pharmaceutique est obtenue dans une pluralité d'itérations, dans lequel dans chacune de la pluralité d'itérations, (i) une pluralité de points de frontière pour chacun de la pluralité de groupes de prix prédéfinis pour chacun de la pluralité de médicaments sont identifiés et ordonnés et sont déplacés vers un groupe de prix adjacent un par un sur la base de l'ordre jusqu'à ce que la qualité du guide de prix pharmaceutique soit maximisée, et (ii) des périodes de temps spécifiques à chacun de la pluralité de médicaments sont modifiées jusqu'à ce que la qualité du guide de prix pharmaceutique soit maximisée, dans lequel la qualité du guide de prix pharmaceutique est mesurée à chaque déplacement du point de prix et à chaque changement de période de temps spécifique à chacun de la pluralité de médicaments en exécutant une analyse de correspondance avec un ensemble mis à jour de groupes de prix obtenus en raison de chaque déplacement du point de prix vers le groupe de prix adjacent, dans lequel le groupe de prix adjacent est décidé sur la base d'un moyen de groupe de prix, dans lequel le déplacement du point de prix pour chaque médicament vers des groupes de prix adjacents entraîne des changements dans la fréquence globale de chaque entité pour chaque groupe de prix et les changements dans la fréquence globale conduisent à une entrée dynamique pour une analyse de correspondance et des changements dans une matrice de correspondance si une qualité mesurée ne change pas sur des itérations consécutives avec le déplacement du point de prix pour un médicament vers des groupes de prix adjacents ; et

la réalisation (218), via les un ou plusieurs processeurs matériels, d'une sélection d'entité spécifique à une application en utilisant le guide de prix pharmaceutique.

2. Procédé selon la revendication 1, dans lequel les données d'entrée comprennent une ou plusieurs parmi a) des informations liées à un médicament, b) des informations liées à une pharmacie, et c) des informations liées à une assurance.

3. Procédé selon la revendication 1, dans lequel le nombre de groupes de prix est prédéfini et fixé à quatre pour permettre de générer une ou plusieurs parmi des vues bidimensionnelles ou tridimensionnelles.

4. Procédé selon la revendication 1, dans lequel la matrice de dimensions de correspondance est estimée en appliquant une analyse de correspondance en utilisant la fréquence globale pour la pluralité d'entités pour chacun de la pluralité de groupes de prix prédéfinis.

5. Procédé selon la revendication 1, dans lequel pour chacune de la pluralité d'entités sélectionnées pour l'application, la qualité mesurée du guide de prix pharmaceutique est obtenue à partir (a) d'une somme de contributions de variance par toutes les dimensions de l'analyse de correspondance, et (b) d'une valeur de charge maximale pour l'entité dans l'une quelconque des dimensions de l'analyse de correspondance.

6. Procédé selon la revendication 1, dans lequel la grandeur d'interrelation entre la pluralité d'entités est extraite en appliquant une pluralité de fonctions trigonométriques sur l'ensemble de dimensions, dans lequel la pluralité de fonctions trigonométriques comprend une distance entre un centroïde avec des coordonnées $(X_m, Y_m)$ et une entité avec des coordonnées $(X_n, Y_n)$.

7. Système (100), comprenant :

un ou plusieurs processeurs matériels (102) ;
une interface de communication (112) ; et
une mémoire (104) stockant une pluralité d'instructions, dans lequel la pluralité d'instructions amènent les un ou plusieurs processeurs matériels à :

recevoir un ensemble d'informations associées à chacun d'une pluralité de médicaments pendant une période de temps prédéfinie, en tant que données d'entrée, dans lequel les données d'entrée contenues dans une base de données externe sont périodiquement mises à jour et de nouvelles données sont ajoutées dans la base de données externe, et des données existantes sont modifiées tandis que des données non utiles sont supprimées de la base de données externe ;

générer une pluralité de groupes de prix prédéfinis pour chacun de la pluralité de médicaments, dans lequel la pluralité de groupes de prix prédéfinis pour chacun de la pluralité de médicaments sont générés en regroupant l'ensemble d'informations dans les données d'entrée à un niveau de transaction pendant une période de temps dynamique spécifique à chacun de la pluralité de médicaments ;

mettre en correspondance chacun de la pluralité de groupes de prix prédéfinis de chacun de la pluralité de médicaments avec au moins l'une d'une pluralité d'entités et calculer la fréquence de chacune de la pluralité d'entités par rapport à chaque groupe de prix parmi la pluralité de groupes de prix prédéfinis pour chacun de la pluralité de médicaments pendant la période de temps dynamique spécifique à chacun de la pluralité de médicaments, dans lequel la période de temps dynamique fait référence à une période de temps particulière qui est choisie par un utilisateur et des informations correspondant à la période de temps particulière à partir des données d'entrée forment un ensemble d'informations qui est regroupé par le système ;

agréger la fréquence de chacune de la pluralité d'entités pour chacun de la pluralité de groupes de prix prédéfinis pour chacun de la pluralité de médicaments pour dériver une fréquence globale pour chacune de la pluralité d'entités pour chacun de la pluralité de groupes de prix prédéfinis ;

estimer une matrice de dimensions de correspondance sur la base de la fréquence globale de la pluralité d'entités pour chacun de la pluralité de groupes de prix prédéfinis, dans lequel la matrice de dimensions de correspondance comprend un ensemble de dimensions représentant la direction de chacune de la pluralité d'entités par rapport à d'autres entités parmi la pluralité d'entités ;

extraire une grandeur d'interrelation entre la pluralité d'entités en utilisant l'ensemble de dimensions dans la matrice de dimensions de correspondance ;

former un guide de prix pharmaceutique sur la base de la grandeur d'interrelation entre la pluralité d'entités ;

maximiser une qualité mesurée du guide de prix pharmaceutique pour chacune de la pluralité d'entités en temps réel, dans lequel la qualité mesurée est maximisée par (i) auto-ajustement du point de prix à travers la pluralité de groupes de prix prédéfinis pour chacun de la pluralité de médicaments pour chacune de la pluralité d'entités en temps réel, (ii) auto-ajustement de la période de temps spécifique à chacun de la pluralité de médicaments, et (iii) considération d'une entité sélectionnée parmi la pluralité d'entités en temps réel et d'une distribution de fréquence de l'entité sélectionnée à travers la pluralité de groupes de prix prédéfinis,

dans lequel la maximisation de la qualité mesurée du guide de prix pharmaceutique est obtenue dans une pluralité d'itérations, dans lequel dans chacune de la pluralité d'itérations, (i) une pluralité de points de frontière pour chacun de la pluralité de groupes de prix prédéfinis pour chacun de la pluralité de médicaments sont identifiés et ordonnés et sont déplacés vers un groupe de prix adjacent un par un sur la base de l'ordre jusqu'à ce que la qualité du guide de prix pharmaceutique soit maximisée, et (ii) des périodes de temps spécifiques à chacun de la pluralité de médicaments sont modifiées jusqu'à ce que la qualité du guide de prix pharmaceutique soit maximisée, dans lequel la qualité du guide de prix pharmaceutique est mesurée à chaque déplacement du point de prix et à chaque changement de période de temps spécifique à chacun de la pluralité de médicaments en exécutant une analyse de correspondance avec un ensemble mis à jour de groupe de prix obtenu en raison de chaque déplacement du point de prix vers le groupe de prix adjacent, dans lequel le groupe de prix adjacent est décidé sur la base d'un moyen de groupe de prix, dans lequel le déplacement du point de prix pour chaque médicament vers des groupes de prix adjacents entraîne des changements de la fréquence globale de chaque entité pour chaque groupe de prix et les changements de la fréquence globale conduisent à une entrée dynamique pour une analyse de correspondance et des changements d'une matrice de correspondance si une qualité mesurée ne change pas sur des itérations consécutives avec le déplacement du point de prix pour un médicament vers des groupes de prix adjacents ; et

réaliser une sélection d'entité spécifique à une application en utilisant le guide de prix pharmaceutique.

8. Système selon la revendication 7, dans lequel les un ou plusieurs processeurs matériels sont configurés pour recevoir une ou plusieurs parmi a) des informations liées à un médicament, b) des informations liées à une pharmacie, et c) des informations liées à une assurance, en tant que données d'entrée.

9. Système selon la revendication 7, dans lequel les un ou plusieurs processeurs matériels sont configurés pour prédéfinir et fixer le nombre de groupes de prix à quatre pour permettre de générer une ou plusieurs parmi des vues bidimensionnelles ou tridimensionnelles.

**10.** Système selon la revendication 7, dans lequel les un ou plusieurs processeurs matériels sont configurés pour estimer la matrice de dimensions de correspondance en appliquant une analyse de correspondance en utilisant la fréquence globale pour la pluralité d'entités pour chacun de la pluralité de groupes de prix prédéfinis.

**11.** Système selon la revendication 7, dans lequel les un ou plusieurs processeurs matériels sont configurés pour obtenir la qualité mesurée du guide de prix pharmaceutique pour chacune de la pluralité d'entités à partir (a) d'une somme de contributions de variance par toutes les dimensions de l'analyse de correspondance, et (b) d'une valeur de charge maximale pour l'entité dans l'une quelconque des dimensions de l'analyse de correspondance.

**12.** Système selon la revendication 7, dans lequel les un ou plusieurs processeurs matériels sont configurés pour extraire la grandeur d'interrelation entre la pluralité d'entités en appliquant une pluralité de fonctions trigonométriques sur l'ensemble de dimensions, dans lequel la pluralité de fonctions trigonométriques comprend une distance entre un centroïde avec des coordonnées $(X_m, Y_m)$ et une entité avec des coordonnées $(X_n, Y_n)$.

**13.** Un ou plusieurs supports de stockage d'informations lisibles par machine non transitoires comprenant une ou plusieurs instructions qui, lorsqu'elles sont exécutées par un ou plusieurs processeurs matériels, amènent :

la réception d'un ensemble d'informations associées à chacun d'une pluralité de médicaments pendant une période de temps prédéfinie, en tant que données d'entrée, dans lequel les données d'entrée contenues dans une base de données externe sont périodiquement mises à jour et de nouvelles données sont ajoutées dans la base de données externe, et des données existantes sont modifiées tandis que des données non utiles sont supprimées de la base de données externe ;

la génération d'une pluralité de groupes de prix prédéfinis pour chacun de la pluralité de médicaments, dans lequel la pluralité de groupes de prix prédéfinis pour chacun de la pluralité de médicaments sont générés en regroupant l'ensemble d'informations dans les données d'entrée à un niveau de transaction pendant une période de temps dynamique spécifique à chacun de la pluralité de médicaments ;

la mise en correspondance de chacun de la pluralité de groupes de prix prédéfinis de chacun de la pluralité de médicaments avec au moins l'une d'une pluralité d'entités et le calcul de la fréquence de chacune de la pluralité d'entités par rapport à chaque groupe de prix parmi la pluralité de groupes de prix prédéfinis pour chacun de la pluralité de médicaments pendant la période de temps dynamique spécifique à chacun de la pluralité de médicaments, dans lequel la période de temps dynamique fait référence à une période de temps particulière qui est choisie par un utilisateur et des informations correspondant à la période de temps particulière à partir des données d'entrée forment un ensemble d'informations qui est regroupé par le système ;

l'agrégation de la fréquence de chacune de la pluralité d'entités pour chacun de la pluralité de groupes de prix prédéfinis pour chacun de la pluralité de médicaments pour dériver une fréquence globale pour chacune de la pluralité d'entités pour chacun de la pluralité de groupes de prix prédéfinis ;

l'estimation d'une matrice de dimensions de correspondance sur la base de la fréquence globale de la pluralité d'entités pour chacun de la pluralité de groupes de prix prédéfinis, dans lequel la matrice de dimensions de correspondance comprend un ensemble de dimensions représentant la direction de chacune de la pluralité d'entités par rapport à d'autres entités parmi la pluralité d'entités ;

l'extraction d'une grandeur d'interrelation entre la pluralité d'entités en utilisant l'ensemble de dimensions dans la matrice de dimensions de correspondance ;

la formation d'un guide de prix pharmaceutique sur la base de la grandeur d'interrelation entre la pluralité d'entités ;

la maximisation d'une qualité mesurée du guide de prix pharmaceutique pour chacune de la pluralité d'entités en temps réel, dans lequel la qualité mesurée est maximisée par (i) l'auto-ajustement d'un point de prix à travers la pluralité de groupes de prix prédéfinis pour chacun de la pluralité de médicaments, (ii) l'auto-ajustement d'une période de temps spécifique à chacun de la pluralité de médicaments, et (iii) la prise en compte d'une entité sélectionnée parmi la pluralité d'entités en temps réel et d'une distribution de fréquence de l'entité sélectionnée à travers la pluralité de groupes de prix prédéfinis, dans lequel la maximisation de la qualité mesurée du guide de prix pharmaceutique est obtenue dans une pluralité d'itérations, dans lequel dans chacune de la pluralité d'itérations, (i) une pluralité de points de frontière pour chacun de la pluralité de groupes de prix prédéfinis pour chacun de la pluralité de médicaments sont identifiés et ordonnés et sont déplacés vers un groupe de prix adjacent un par un sur la base de l'ordre jusqu'à ce que la qualité du guide de prix pharmaceutique soit maximisée, et (ii) des périodes de temps spécifiques à chacun de la pluralité de médicaments sont modifiées jusqu'à ce que la qualité du guide de prix pharmaceutique soit maximisée, dans lequel la qualité du guide de prix pharmaceutique est mesurée à chaque déplacement du point de prix et à chaque changement de période de temps spécifique à chacun de la pluralité de médicaments en exécutant une analyse de correspondance avec un ensemble mis à jour de groupes de prix obtenus en raison de chaque déplacement du point de prix vers le groupe de prix adjacent, dans lequel le groupe de prix adjacent est décidé sur la base d'un moyen de groupe de prix, dans lequel le déplacement du point de prix pour chaque médicament vers des groupes de prix adjacents entraîne des changements de la fréquence globale de chaque entité pour chaque groupe de prix et les changements de la fréquence globale conduisent à une entrée dynamique pour une analyse de correspondance et des changements dans une matrice de correspondance si une qualité mesurée ne change pas sur des itérations consécutives avec le déplacement du point de prix pour un médicament vers des groupes de prix adjacents ; et réaliser une sélection d'entité spécifique à une application en utilisant le guide de prix pharmaceutique.

EP 4 343 659 B1

FIG. 1

21

202

receiving, via one or more hardware processors, a set of
information associated with each of a plurality of drugs for a
predefined period of time, as input data

204

generating a plurality of predefined price groups for each of the
plurality of drugs, via the one or more hardware processors,
wherein the plurality of predefined price groups for each of the
plurality of drugs are generated by grouping the set of
information in the input data at a transaction level for a dynamic
time period specific to each of the plurality of drugs

206

mapping, via the one or more hardware processors, each of the
of the plurality of predefined price groups of each of the
plurality of drugs to at least one of a plurality of entities and
calculating frequency of each of the plurality of entities against
each price group from among the plurality of predefined price
groups for each of the plurality of drugs for the dynamic time
period specific to each of the plurality of drugs

208

aggregating, via the one or more hardware processors, frequency
of each of the plurality of entities for each of the plurality of
predefined price groups for each of the plurality of drugs to
derive an overall frequency for each of the plurality of entities
for each of the plurality of predefined price groups

A

FIG. 2A

200

A

estimating, via the one or more hardware processors, a
correspondence dimension matrix based on the overall frequency of
the plurality of entities for each of the plurality of predefined price
groups, wherein the correspondence dimension matrix comprises a
set of dimensions representing direction of each of the plurality of
entities in relation to other entities among the plurality of entities

210

extracting, via the one or more hardware processors, a magnitude of
interrelationship between the plurality of entities using the set of
dimensions in the correspondence dimension matrix

212

forming, via the one or more hardware processors, a pharmaceutical
pricing guide based on the magnitude of interrelationship between
the plurality of entities

214

maximizing, via the one or more hardware processors, a measured
quality of the pharmaceutical pricing guide for each of the plurality
of entities in real time, wherein the measured quality is maximized
by (i) self-adjusting of price point across the plurality of predefined
price groups for each of the plurality of drugs, (ii) self-adjusting
time period specific to each of the plurality of drugs, and by (iii)
considering a selected entity from among the plurality of entities in
real time and a frequency distribution of the selected entity across
the plurality of predefined price groups

216

performing, via the one or more hardware processors, an application
specific entity selection using the pharmaceutical pricing guide

218

FIG. 2B

200

| Drug Specific Dynamic Period | Drug | Drug Specific Dynamic unit price range ($) | Predefined Price Group | Frequency of Pharma players (Entities) Under Each Price Group for all drugs | | | | | | | | | | | | |
|---|---|---|---|---|---|---|---|---|---|---|---|---|---|---|---|---|
| | | | | PBM_1 | PBM_2 | ....... | PBM_ | Insurer_1 | ....... | Insurer_ | Insurance Plan_ | ....... | Brand_1 | ....... | ....... | ....... |
| Last 6 months | Drug_1 | $ 2.1 - $ 2.8 | Low | | | | | | | | | | | | | |
| | | $ 2.9 - $ 4.0 | Medium | | | | | | | | | | | | | |
| | | $ 4.1 - $ 5.5 | High | | | | | | | | | | | | | |
| | | $ 5.6 - $ 7.2 | Very High | | | | | | | | | | | | | |
| Last 30 months | Drug_2 | $ 7.1 - $ 7.9 | Low | | | | | | | | | | | | | |
| | | $ 8.0 - $9.0 | Medium | | | | | | | | | | | | | |
| | | $ 9.1 - $ 10.5 | High | | | | | | | | | | | | | |
| | | $ 10.6 - $ 12.9 | Very High | | | | | | | | | | | | | |
| ..... | Drug_ | .... | Low | | | | | | | | | | | | | |
| | | ... | Medium | | | | | | | | | | | | | |
| | | ... | High | | | | | | | | | | | | | |
| | | ... | Very High | | | | | | | | | | | | | |
| ...... | Overall | | Low | -------- | | | | | | | | | | | | |
| | | | Medium | -------- | | | | | | | | | | | | |
| | | | High | -------- | | | | | | | | | | | | |
| | | | Very High | -------- | | | | | | | | | | | | |

FIG. 3A

Correspondence dimension matrix

| Pharma players | Function_1 | Function_2 | Function_3 |
|---|---|---|---|
| PBM_1 | 0.4 | 0.9 | 0.8 |
| PBM_2 | -0.8 | -0.9 | -0.7 |
| PBM_3 | -0.9 | -0.7 | 0.9 |
| PBM_4 | 0.7 | 0.9 | 0.7 |
| PBM_5 | 0.8 | 0.6 | 0.75 |
| PBM_6 | -0.85 | -0.95 | -0.75 |
| PBM_7 | -0.95 | -0.75 | 0.85 |
| PBM_8 | 0.65 | 0.75 | 0.65 |

| Tick | Pharma Players | Tick | Pharma players |
|---|---|---|---|
| ✓ | PBMs | | PBM_1 |
| | Insurer | | PBM_2 |
| | Insurance Plans | | PBM_3 |
| | Brands | | PBM_4 |
| | | ✓ | PBM_5 |
| | | | PBM_6 |
| | | | PBM_7 |
| | | | PBM_8 |
| | | | Insurer_1 |
| | | | Insurer_2 |
| | | | Insurer_3 |
| | | | Insurance Plan_1 |
| | | | Insurance Plan_2 |
| | | | Insurance Plan_3 |
| | | | Brand_1 |
| | | | Brand_2 |
| | | | Brand_3 |
| | | | ....... |
| | | | ....... |

FIG. 3B

PBM_x
A (0.4,0.9)

(0.8,0.6)
C
PBM_y

θ

B (0,0)

Dimension 2

Dimension 1

-1        0        1

A= (X1, Y1)
Example (0.4,0.9)

B= (X2, Y2)
AB = SQRT { (X1-X2)^2+(Y1-Y2)^2}
Cos θ = BC/AB

FIG. 3C

FIG. 3D

FIG. 3E

FIG. 3F

FIG. 3G

FIG. 3H

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202221054667
- US 20140358578 A1
- US 2020105392 A